# EUROPÄISCHE PATENTANMELDUNG

(11) **EP 3 162 307 A1**
(43) Veröffentlichungstag der Anmeldung: **03.05.2017**
(21) Anmeldenummer: 15192411.5
(22) Anmeldetag: 30.10.2015
(51) Int. Cl.: A61B 17/16, A61B 90/00, A61B 17/29

(54) **CHIRURGISCHES INSTRUMENT**

(71) Anmelder: Heinemann, Norbert, 78549 Spaichingen (DE)
(72) Erfinder: Heinemann, Norbert, 78549 Spaichingen (DE)
(74) Vertreter: Wagner, Kilian

(57) **Zusammenfassung**

Die Erfindung betrifft ein chirurgisches Instrument, insbesondere Arthroskopie-Stanze, mit einem Maul (22), umfassend ein feststehendes Maulteil (3) und ein relativ zu dem feststehenden Maulteil (3) durch Verschenken eines verschwenkbaren Griffteils (6) in einer Kulissenführung (26) entlang einer gekrümmten Führungsbahn zwischen einer Schließposition und einer Offenposition verschiebbares bewegliches Maulteil (10).

## Beschreibung

Die Erfindung betrifft ein chirurgisches Instrument, insbesondere eine Arthroskopie-Stanze, mit einem Maul, umfassend ein feststehendes Maulteil und ein relativ zu dem feststehenden Maulteil durch Verschwenken eines verschwenkbaren Griffteils in einer Kulissenführung entlang einer gekrümmten Führungsbahn zwischen einer Schließposition und einer Offenposition verschiebbares bewegliches Maulteil.

Arthroskopie-Stanzen werden eingesetzt, um knorpelige Gelenkanteile zu entfernen oder sehr harte Muskeln bzw. Muskelstränge, wie den Miniskusmuskel. Daher sind die auf das chirurgische Instrument und insbesondere die Lagerung des beweglichen Maulteils wirkenden Kräfte groß. Um diesem Problem zu begegnen wurde in der US 4,712,545 ein chirurgisches Instrument vorgeschlagen, bei welchem das bewegliche Maulteil relativ zu dem feststehenden Maulteil in einer Kulissenführung entlang einer gekrümmten Führungsbahn verschiebbar ist, so dass die wirkenden Kräfte nicht nur punktuell an einer Gelenkachse abgestützt werden, sondern großflächig an einer Kulissennutseitenwand. Hierdurch ist es möglich, die üblicherweise beim Einsatz von Arthroskopie-Stanzen auftretenden großen Kräfte aufzunehmen, ohne eine Beschädigung der Belagerung des beweglichen Maulteils zu riskieren, insbesondere auch bei kleineren Ausführungen von Arthroskopie-Stanzen, bei denen für die Aufnahme der wirkenden Kräfte vergleichsweise wenig Material zur Verfügung steht. Das aus der vorgenannten US-Schrift bekannte chirurgische Instrument ist als abgewandeltes Rohrschaftinstrument ausgebildet, mit einem halboffenen, feststehenden Rohrteil und einer in der Rohröffnung aufgenommenen Schubstange, die über eine weitere Kulissenführung mit dem bewegbaren Maulteil zusammenwirkt. Durch diese ist zwar eine stabile Lagerung der Zugstange im bewegbaren Maulteil gewährleistet. Eine Zerlegung des Instrumentes zu Reinigungszwecken ist jedoch nicht möglich - um das Instrument zerlegen zu können, wird ein Werkzeugbauer mit entsprechendem Werkzeug benötigt, so dass eine Zerlegung im praktischen Einsatz nicht realisierbar ist. Darüber hinaus muss die Zugstange mangels translatorischer Führung am Rohrteil mit einem vergleichsweise großen Durchmesser realisiert werden, was insbesondere bei dem Instrument für filigrane Einsätze von Nachteil ist. Zudem wird als nachteilig empfunden, dass sich bauartbedingt während der Öffnungs- und Schließbewegung des Maulteils der Abstand zwischen der Zugstange und dem oben offenen Rohr verändert - hier besteht die Gefahr des Einklemmens von Material, was die Funktionsweise des chirurgischen Instrumentes während der Operation beeinträchtigen kann.

Grundsätzlich bekannt sind Schiebeschaftinstrumente mit einem um eine Drehachse verschwenkbares Schiebeteil, bei denen ein bewegliches Maulteil um einen feststehenden Stift verschwenkbar ist. Die Kraftaufnahme des Gelenkstiftes zur verschwenkbaren Lagerung des beweglichen Maulteils am Schaft ist begrenzt. Derartige Schiebeschaftinstrumente sind beispielsweise in der DE 2006 043 970 A1, DE 2 992 458 U1 oder der US 5,961,531 A beschrieben. Bei den bekannten Schiebeschaftinstrumenten ist der Schiebeschaft nach Überführung aus einer Arbeitsstellung in eine Reinigungsstellung um eine in Querrichtung des Instrumentes verlaufende Drehachse verschwenkbar. Ein hinsichtlich der Reinigbarkeit verbessertes Schiebeschaftteil wurde vom Anmelder in der EP 2 213 254 B1 beschrieben - hier ist das Schiebeteil um eine vertikale Drehachse verdrehbar, wobei jedoch auch hier der Nachteil besteht, dass nur begrenzt große Kräfte zwischen Schiebeteil und beweglichen Maulteil übertragen werden können.

Der Erfindung liegt daher die Aufgabe zugrunde, ein verbessertes chirurgisches Instrument, insbesondere eine Arthroskopie-Stanze anzugeben, die sich trotz der Realisierung eines kulissenführungs-gelagerten beweglichen Mauteils zur Übertragung großer Kräfte vereinfacht reinigen lässt.

Diese Aufgabe wird mit einem chirurgischen Instrument, insbesondere einer Arthroskopie-Stanze, mit den Merkmalen des Anspruchs 1 gelöst, d.h. bei einem gattungsgemäßen chirurgischen Instrument dadurch, dass das chirurgische Instrument nicht als Rohr-Schaft-Instrument, sondern als Schiebeschaft-Instrument ausgebildet ist, umfassend einen dem feststehenden Maul zugeordneten, bevorzugt einteilig mit diesem ausgebildeten, Schaft und ein durch Verschwenken des Griffteils relativ zu dem Schacht translatorisch, insbesondere senkrecht zur translatorischen Verstellrichtung äquidistant, entlang des Schaftes längsverschiebbares Schiebeteil, welches zwischen einer Arbeitsstellung, in der es an dem Schaft längsverschieblich in einer Führung, insbesondere einer Führungsnut, geführt und gelenkig mit dem beweglichen Maulteil zum Verstellen des Maulteils zwischen der Schließposition und der offenen Position bei einer geführten Verschiebebewegung des Schiebeteils gekoppelt ist und einer Reinigungsstellung, in der das Schiebeteil von dem beweglichen Maulteil entkoppelt, aus der Führung (relativ zum Schacht) befreit und um eine Drehachse relativ zu dem Schaft sowie relativ zu dem beweglichen Maulteil verschwenkbar ist, verstellbar ist und dass dem Schiebeteil manuell betätigbare Verriegelungsmittel zugeordnet sind, mit denen das Schiebeteil in seiner Arbeitsstellung gegen ein unbeabsichtigtes Überführen in die Reinigungsstellung sicherbar ist.

Vorteilhafte Weiterbildungen der Erfindung sind in den Unteransprüchen angegeben. In den Rahmen der Erfindung fallen sämtliche Kombinationen aus zumindest zwei von in der Beschreibung, den Ansprüchen und/oder den Figuren offenbarten Merkmalen.

Der Erfindung liegt ein ganzes Maßnahmenpaket zur Lösung der der Erfindung zugrundeliegenden Aufgabe zugrunde. Zunächst ist erfindungsgemäß vorgesehen, das chirurgische Instrument als Schiebeschaftinstrument auszubilden, welches also ein Schiebeteil umfasst, welches relativ zu einem feststehenden, insbesondere nicht rohr- oder teilrohrförmigen, Schaft verschiebbar ist, wobei das Schiebeteil während seiner mittels des verschwenkbaren Griffteils aktuierten Verschiebebewegung längssverschieblich am Schaft geführt ist, insbesondere derart, dass der senkrecht zur Schaftbreite und Schaftlänge gemessene Abstand zwischen Schiebeteil und Schaft bei der Verstellbewegung konstant bleibt, da das Schiebeteil aufgrund der Führung am Schaft gefangen ist.

Bevorzugt ist dieser Abstand minimal oder nicht vorhanden und das Schaftteil gleitet direkt auf den Schaft. Durch diese Maßnahmen wird bereits ein erstes Ziel erreicht, das Schiebeteil mit einer vergleichsweise geringen Durchmesser bzw. einer geringer Materialstärke realisieren zu können.

Des Weiteren eröffnet die Realisierung eines Schiebeschaftinstrumentes im Gegensatz zu einem Rohrschaftinstrument die Möglichkeit, in erfindungsgemäßer Weise das Schiebeteil lösbar gelenkig mit dem kulissengeführten beweglichen Maulteil zu verbinden, derart, dass eine werkzeuglose Entkopplung des Schiebeteils von dem beweglichen Maulteil möglich ist, nachdem dem Schiebeteil zumindest mittelbar zugeordnete Verriegelungsmittel entriegelt wurden. Die Verriegelungsmittel sind notwendig, um zu verhindern, dass das Schiebeteil versehentlich bzw. unbeabsichtigt aus einer Arbeitsstellung in eine Reinigungsstellung überführbar ist, in welcher das Schiebeteil im Gegensatz zu einer Zugstange eines Rohrschaftinstrumentes relativ zum Schaft sowie zum beweglichen Maulteil verschwenkbar ist, um den Bereich zwischen Schiebeteil und Schaft erleichtert reinigen zu können.

In der Arbeitsstellung dagegen, ist die axiale, translatorische Verstellbarkeit des Schiebeteils relativ zum Schaft zwischen zwei Extrempositionen begrenzt, die die Schließposition und Offenposition des Mauls definieren, wobei die Offenposition vorzugsweise definiert wird von den Verriegelungsmitteln, welche beispielsweise zu diesem Zweck die Schwenkbewegung des Griffteils, d.h. den Schwenkwinkel des Griffteils und damit wiederum den axialen Verstellweg des Schiebeteils begrenzen, wodurch dann im Ergebnis auch die Verschiebeposition des beweglichen Maulteils in der Kulissenführung begrenzt ist. Wesentlich ist also, dass das Schiebeschaftinstrument ausgebildet ist, um eine werkzeuglose Entkopplung von Schiebteil und beweglichem Maulteil zu ermöglichen, dass also eine entsprechende Wirkverbindung werkzeuglos geöffnet werden kann, mit dem Ergebnis, dass der Schiebeschaft um eine Drehachse relativ zum beweglichen Maulteil und dem Schaft verschwenkt werden kann.

Besonders zweckmäßig, insbesondere im Hinblick auf eine reduzierte Teilezahl sowie eine erhöhte Robustheit und Stabilität des chirurgischen Instrumentes ist es, das feststehende Maulteil als Bestandteil des Schaftes, also einteilig mit diesem auszubilden, wobei es alternativ grundsätzlich auch denkbar ist, das feststehende Maulteil als separates Bauteil auszubilden und, insbesondere dauerhaft fest mit dem Schaft zu verbinden.

Insgesamt schafft die Erfindung also ein chirurgisches Instrument, bei dem erstmals aufgrund der Kulissenlagerung des beweglichen Maulteils relativ zum feststehenden Maulteil, insbesondere unmittelbar am Schaft große Stanzkräfte (großflächig) übertragen werden können und trotzdem aufgrund der speziellen Ausbildung des chirurgischen Instruments als Schiebeschaftinstrument, aufweisend ein werkzeuglos lösbar mit dem beweglichen Maulteil koppelbares Schiebeteil, welches bei seiner Verschiebebewegung relativ zu dem Schaft in einer Führung, insbesondere einer Führungsnut im Schaft, geführt ist, geringe Materialstärken realisieren zu können und zudem eine gute Reinigbarkeit sicher zu stellen, aufgrund der verschwenkbaren Anordnung des Schiebeteils um eine Drehachse, insbesondere nach werkzeuglosem Lösen der Wirkverbindung zwischen Schiebeteil und bewegbarem Mauteil. Wie eingangs bereits angedeutet, ist das Schiebeteil in seiner Arbeitsstellung am Schaft bzw. einer dieser zugeordneten Führung, insbesondere einer im Schaft vorgesehenen Führungsnut gefangen, insbesondere derart, dass der senkrecht zur Längserstreckung des Schaftes sowie senkrecht zur Breitenerstreckung des Schaftes, und, wie später noch erläutert werden wird, vorzugsweise parallel zur Drehachse gemessene Abstand (Höhenabstand) des Schiebeteils zum Schaft in der Schließposition und der Offenposition des beweglichen Maulteils sowie während der Verstellbewegung zwischen diesen Positionen gleich ist. Anders ausgedrückt verändert sich der sich der vorgenannte Abstand, insbesondere über die gesamte Längserstreckung des Schiebeteils zum Schaft, nicht bei einer Verstellung des beweglichen Maulteils während sich das Schiebeteil in seiner Arbeitsstellung befindet und die Verriegelungsmittel verriegelt sind. Alternativ ist während der gesamten Verstellbewegung kein vorbeschriebener Abstand vorhanden und das Schiebeteil gleitet auf den Schacht.

Wie erwähnt ist das erfindungsgemäße chirurgische Instrument in einer bevorzugten Ausführung als Stanze, insbesondere als Arthroskopie-Stanze ausgebildet - hierauf ist die Erfindung jedoch ausdrücklich nicht beschränkt. So kann das Maul bzw. Arbeitsende beispielweise auch als Schere mit einem beweglichen und einem feststehenden Scherenteil oder als Fasszange mit einem beweglichen und einem feststehenden Zangenteil ausgebildet werden.

Besonders zweckmäßig ist es, wenn die Kopplung/Wirkverbindung zwischen Schiebeteil und beweglichem Maulteil so realisiert ist, dass eine Kopplung und/oder Entkopplung bei parallelisiertem Schiebeteil und Schaft (nach entsprechender Entriegelung der Verriegelungsmittel) durch eine einfache Verschwenkbewegung des beweglichen Griffteils und eine daraus resultierende translatorische Verstellbewegung des Schiebeteils relativ zum beweglichen Maulteil möglich ist, insbesondere derart, dass mindestens ein stiftförmiger Fortsatz des Schiebeteils zum Entkoppeln aus mindestens einer entsprechenden Gelenkaufnahme des beweglichen Schiebeteils herausverstellt bzw. zum Koppeln wieder in diese hineinverstellt ist bzw. verstellbar ist. Bevorzugt ist hierzu die Wirkverbindung zwischen Schiebeteil und beweglichem Maulteil nicht als Kulissenführung, sondern als bewusst einfache Gelenkverbindung ausgestaltet, mit der das bewegliche Maulteil um eine durch den Stiftfortsatz des Schiebeteils verlaufende bzw. von diesem definierte Schwenkachse verdrehbar ist, so dass ein Entlangverschieben des Schiebeteils in einer Kulissenführung zu Zwecken einer einfachen Kopplung und Entkopplung vermieden wird.

Im Hinblick auf die konkrete Ausbildung der Kulissenführung ist es bevorzugt, wenn diese derart gestaltet ist, dass das bewegliche Maulteil während seiner Verschiebebewegung entlang der gekrümmten Führungsbahn eine Teilkreisbahn um eine gedachte Schwenkachse beschreibt, die bevorzugt mit Abstand zum Schiebeschaftteil verläuft, d.h. dieses bevorzugt nicht schneidet.

Besonders zweckmäßig ist es, wenn die Kulissenführung mindestens eine, insbesondere schaftseitig und/oder im feststehenden Maulteil angeordnete gekrümmte, insbesondere teilkreisförmige, die Führungsbahn definierende Kulissennut umfasst, in der ein, insbesondere gekrümmter, bevorzugt mit dem gleichen Krümmungsradius wie die Kulissennut gekrümmter, bevorzugt am beweglichen Maulteil vorgsehener Eingriffsabschnitt entlang der Kulissennutlängserstreckung bei der Bewegung des beweglichen Maulteils zwischen der Schließposition und der Offenposition verschiebbar ist. Bevorzugt verläuft die Kulissenbahn mit Radialabstand konzentrisch zu der gedachten Schwenkachse.

Bevorzugt umfasst die Kulissenführung insgesamt zwei in Richtung der Breiten- bzw. Quererstreckung des chirurgischen Instrumentes beabstandete Kulissenführungspaarungen, jeweils umfassend eine Kulissennut und ein darin verschieblich geführten Eingriffsabschnitt, der sich bevorzugt nicht nur entlang einer in Querrichtung des chirurgischen Instrumentes verlaufenden Anlagelinie an einer Nutwand abstützt, sondern großflächig.

Wie bereits angedeutet, ist es bevorzugt, wenn Schiebeteil und bewegliches Maulteil in der Arbeitsstellung des Schiebeteils nicht über eine Kulissenführung verbunden sind, sondern über eine, insbesondere klassische, Gelenkverbindung, bei der mindestens ein Stiftfortsatz bzw. Stift des Schiebeteils, der sich in Richtung der Breitenerstreckung/Quererstreckung des chirurgischen Instrumentes erstreckt, in eine offene Gelenkvertiefung des Schaftteils eingreift, um somit einfach von diesem entkoppelt zu werden, insbesondere durch eine translatorische Bewegung des Schiebeteils. Um die gelenkige Verbindung im Hinblick auf die maximal übertragbaren Kräfte zu optimieren, insbesondere ausschließlich derart, dass zwischen Schiebeteil und beweglichem Maulteil Kräfte von über 50,96 kpond (500 N), bevorzugt über 61,18 kpond (600 N), noch weiter über 71,38 kpond (700 N), übertragbar sind ist es besonders zweckmäßig, wenn sich das Schiebeteil nicht mit nur einem einzigen Gelenkstift am beweglichen Maulteil abstützt, sondern wenn an dem Schiebeteil zwei ineinander entgegengesetzte Richtungen weisende, einzeilig mit dem Schiebeteil ausgebildete oder daran festgelegte (separate) Stiftfortsätze vorgesehen sind und jedem Stiftfortsatz eine Gelenkvertiefung dem beweglichen Maulteil zugeordnet ist. Hierdurch können Hebelwege verkürzt und eine gleichmäßige Abstützung bzw. Kraftübertragung gewährleistet werden.

Besonders zweckmäßig ist es, wenn das Schiebteil in der Arbeitsposition axial, d.h. in Richtung der Längserstreckung des Schaftes in eine nach proximal hinten offene Aussparung (des beweglichen Maulteils, also in das bewegliche Maulteil hinein) eingreift, wobei diese Aussparung von zwei in Richtung der Breitenerstreckung des Instrumentes beabstandete, insbesondere parallele Wandabschnitte begrenzt ist, die bevorzugt, insbesondere in einem oberen Bereich, jeweils eine Gelenkvertiefung zur Aufnahme eines vorerwähnten Fortsatzes des Schiebeteils aufweisen.

Insgesamt hat es sich als vorteilhaft herausgestellt, wenn das bewegliche Maulteil mit dem feststehenden Maulteil in der Offenstellung einen Öffnungswinkel, insbesondere von kleiner als 90°, aufspannt, der, insbesondere mittelbar durch unmittelbares Zusammenwirken der Verriegelungsmittel mit dem verschwenkbaren Griffteil, von den Verriegelungsmitteln begrenzt ist, und dass das bewegliche Maulteil, insbesondere nach Entriegeln der Verriegelungsmittel, zum Entkoppeln von dem Schiebeschaft in eine Entriegelungs(Winkel)Position verstellbar ist, in der das bewegliche Maulteil und das feststehende Maulteil einen Entriegelungswinkel aufspannen, insbesondere von größer als 90°, der größer ist als der Öffnungswinkel. Anders ausgedrückt kann bevorzugt das Maul durch Verstellen des Schiebeteils nach dessen Entriegelung weiter geöffnet werden als in der Arbeitsstellung des Schiebeteils, um somit ein, insbesondere translatorisches Herausverstellen des Schiebeteils zu ermöglichen, insbesondere um das Schiebeteil danach in seiner Reinigungsstellung zu verschwenken.

Denkbar ist es jedoch auch, insbesondere bei einer Anordnung der Drehachse des Schiebeteils in Richtung der Breitenerstreckung des Schiebeteils, wenn das Schiebeteil bereits in der Offenstellung oder alternativ in einer dann fakultativ vorgesehen Entriegelungsposition durch Verschwenken um die Drehachse von dem beweglichen Maulteil entkoppelbar ist.

Erfindungsgemäß ist das Schiebeteil in seiner Arbeitsstellung am Schaft in einer Führung gefangen. Bevorzugt umfasst diese Führung, wie ebenfalls bereits erwähnt eine Führungsnut. Diese kann zwar grundsätzlich im Schiebeteil vorgesehen werden und ein Führungsnuteingriffsabschnitt am Schaft, wobei eine umgekehrte Anordnung aufgrund der Materialstärkenverhältnisse bevorzugt ist. Jedenfalls ist es zweckmäßig, diese Führungsnut als hinterschnittene Führungsnut auszubilden. Bei bekannten Schiebeschaftinstrumenten ist zu diesem Zweck die Führungsnut als T-Nut ausgebildet. In Weiterbildung der Erfindung ist nun vorgesehen, dass die Führungsnut nicht als T-Nut ausgebildet ist, zumindest nicht axial durchgängig und zumindest in einem Abschnitt einen benachbart zur Führungsnutöffnung ausgebildeten Hinterschnittraum aufweist, der keinen rechteckigen Querschnitt aufweist, sondern dessen Erstreckung in Richtung der Breitenerstreckung des Instrumentes in einen näher an der Führungsöffnung gelegenen Bereich, insbesondere an die Führungsöffnung angrenzenden Abschnitt, größer ist als in einem weiter von der Führungsöffnung entlang der Hocherstreckung des chirurgischen Instrumentes beabstandeten Abschnitt, um somit an dem die Führungsnut bereitstellenden Bauteil, insbesondere dem Schaft, möglichst viel Material belassen zu können, um große Kräfte aufnehmen zu können. Im Hinblick auf den Übergang zwischen dem breiteren und dem schmaleren Abschnitt des Hinterschnittraums gibt es unterschiedliche Möglichkeiten - im Hinblick - auf eine einfache Fertigung ist es bevorzugt, wenn die Seitenwände hierzu abgeschrägt sind, derart, dass eine trapezförmige Querschnittskontur des Hinterschnittraums resultiert.

Auch die Materialwahl zur Gewährleistung einer langfristig robusten Ausführungsform kann in Weiterbildung der Erfindung optimiert werden. So ist bei einer besonders bevorzugten Ausführungsform das bewegliche Maulteil aus einer anderen Stahllegierung ausgebildet, als das feststehende Bauteil und der Schaft, dahingehend, dass die Stahllegierung des beweglichen Maulteils aus einem zäherem und damit weniger spröden Stahlmaterial auszubilden, als den Schaft und/oder das feststehende Maulteil.

Dies kann erreicht werden, durch eine höhere Rockwellhärte, insbesondere um mindestens 4 HRC höhere Rockwellhärte des Stahlmaterials des feststehenden Maulteils und des Schaftes und/oder um einen höheren Kohlenstoffanteil, insbesondere um ein um mindestens 0,2 % höheren Kohlenstoffanteil dieses Stahlmaterials als das Stahlmaterial der Stahllegierung des beweglichen Maulteils.

Insgesamt ist es von Vorteil, wenn die Rockwellhärte des beweglichen Maulteils aus einem Wertebereich zwischen 45 HRC und 47 HRC und/oder die Rockwellhärte der Stahllegierung des Schaftes und/oder des feststehenden Bauteils aus einem Wertebereich zwischen 55 HRC und 57 HRC gewählt ist.

Insgesamt ist es von Vorteil, wenn das Schiebeteil derart an dem Schaft mittels der Führung gefangen ist, dass das Schiebeteil nach Entriegeln der Entriegelungsmittel nicht unmittelbar verschwenkbar ist, sondern zunächst noch translatorisch ein Stück weit geführt von dem Maul weg in Richtung proximalem Ende des chirurgischen Instrumentes verstellbar ist. Anders ausgedrückt ist es zweckmäßig, wenn das Schiebeteil nach Entriegeln der Verriegelungsmittel translatorisch weiter von dem Maul wegverstellbar ist, insbesondere derart, dass das Schiebeteil, bevor dieses aus der Führung freikommt, um relativ zu dem Schaft und dem Maul verschwenkt werden zu können, ein Stück weit innerhalb der Führung gefangen bleibt bzw. translatorisch von dem Maul weg in der Führung gefangen verstellbar ist, insbesondere über eine Strecke von mindestens 1 mm, bevorzugt zwischen 1 mm und 5mm, ganz besonders bevorzugt zwischen 2 mm und 3 mm. Eine solche Ausführungsform ist insbesondere dann vorteilhaft, wenn, wie später noch erläutert werden wird, die Drehachse, um die das Schiebeteil in der Reinigungsstellung verschwenkbar ist, senkrecht zur Schwenkachse des Griffteils orientiert ist sowie senkrecht zur Längsachse des Schaftes.

Insbesondere bei einer Ausführung, bei der die Drehachse parallel zur Schwenkachse des Griffteils orientiert ist, ist es denkbar, dass auf eine solche gefangene translatorische Verstellung nach Entriegeln der Verriegelungsmittel verzichtet wird, wobei es auch bei einer solchen Ausführungsform vorteilhaft ist, eine weitere, in der Führung gefangene translatorische Verstellbewegung des Schiebeteils vorzusehen, bevor das Schiebeteil aus der Führung freikommt, um dann relativ zum Schaft und dem beweglichen Maulteil verschwenkt werden zu können.

Wie erwähnt, ist es besonders zweckmäßig, wenn das Schiebeteil in seiner Reinigungsstellung um ein sich in Hochrichtung des Instrumentes, d.h. senkrecht zur Längserstreckung sowie senkrecht zur Breitenerstreckung und damit senkrecht zur Orientierung der Schwenkachse des Griffteils orientierte Drehachse verdrehbar angeordnet ist, wobei es dabei noch weiter bevorzugt ist, wenn das Schiebeteil sowohl in der Arbeitsstellung als auch in der Reinigungsstellung und während der Schwenkbewegung relativ zum Schaft unverlierbar am Schaft gesichert ist.

Weitere Vorteile, Merkmale und Einzelheiten der Erfindung ergeben sich aus der nachfolgenden Beschreibung bevorzugter Ausführungsbeispiele sowie anhand der Zeichnungen.

Diese zeigen in:
- Fig. 1: eine teilgeschnittene Seitenansicht eines als Arthroskopie-Stanze in der Form eines Schiebeschaftinstrumentes ausgebildeten chirurgischen Instrumentes, wobei sich das Schiebeteil in einer Arbeitsstellung befindet, in der das Schiebeteil längsverschieblich zum Schaft geführt ist,
- Fig. 2: eine Draufsicht auf das chirurgische Instrument gemäß Fig. 1,
- Fig. 3: eine Draufsicht auf das chirurgische Instrument bei in der Reinigungsstellung befindlichem Schiebeteil, welches zudem relativ zum Schaft sowie zum Maul verschwenkt ist,
- Fig. 4: eine vergrößerte geschnittene Darstellung des distalen Instrumentenendes mit geöffnetem Maul,
- Fig. 5: eine Alleindarstellung des beweglichen Maulteils in einer Längsschnittansicht,
- Fig. 6: eine Draufsicht auf das bewegliche Maulteil gemäß Fig. 5,
- Fig. 7: eine Querschnittsansicht einer Führungsnut zur Führung des Schiebeteils am Schaft.

In den Figuren sind gleiche Elemente und Elemente mit der gleichen Funktion mit den gleichen Bezugszeichen gekennzeichnet.

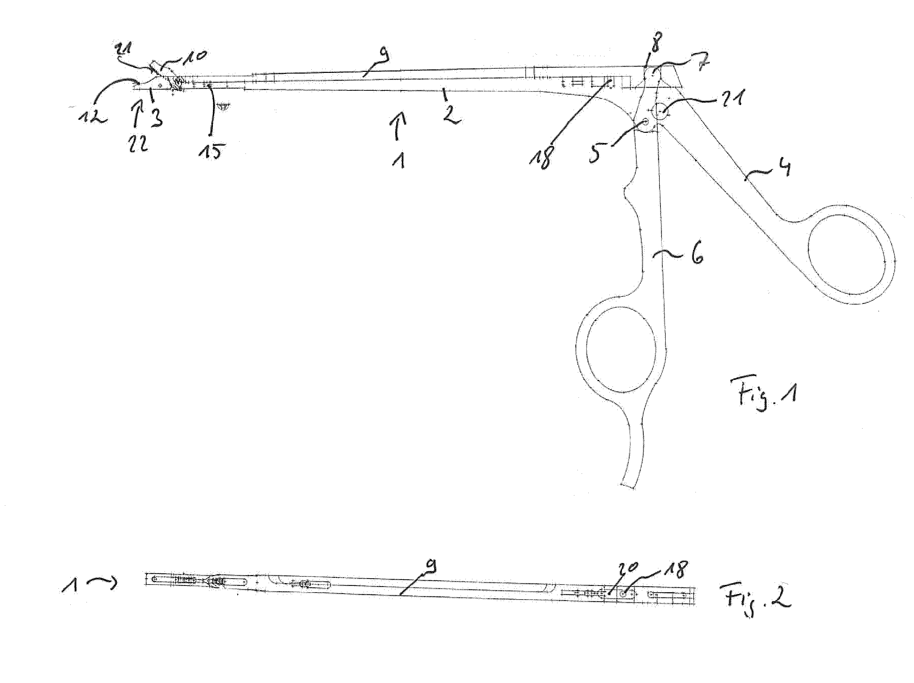

In den Fig. 1 bis 3 ist ein bevorzugtes Ausführungsbeispiel eines erfindungsgemäßen, als Arthroskopie-Stanze ausgebildeten chirurgischen Instrumentes 1 in seiner Gesamtheit gezeigt. Das chirurgische Instrument 1 umfasst einen langgestreckten, feststehenden Schaft 2 mit einem distalen (vorderen) Arbeitsende, welches zum Teil von einem einteilig mit dem Schaft 2 gebildeten feststehenden Maulteil 3 gebildet ist. Einteilig mit dem Schaft 2 ist auch ein hinteres Griffteil 4 (Griffbranche) ausgebildet.

Relativ zu dem feststehenden hinteren (proximalen) Griffteil 4 ist um eine quer zur Längserstreckung des Schaftes 2 verlaufende Schwenkachse 5 ein distales (vorderes) verschwenkbares Griffteil 6 verschwenkbar angeordnet.

Das verschwenkbare (vordere) Griffteil 6 weist ein oberes, näherungsweise kugelkopfförmiges Ende 7 auf, welches in eine seitlich offene Aussparung 8 eines auf den Schaft 2 angeordneten Schiebeteils 9 eingreift. Auf diese Weise ist das Schiebeteil 9 mittels des vorderen, verschwenkbaren Griffteils 6, durch Verschwenken desselben um die Schwenkachse 5 relativ zu dem Schaft 2 entlang der Längserstreckung des Schaftes 2 längsverschieblich antreibbar.

Das Schiebeteil 9 ist lösbar gelenkig mit einem verschwenkbaren Maulteil 10 verbunden, solange sich das Schiebeteil 9 in einer später noch zu erläuternden Arbeitsstellung befindet. Das verschwenkbare Maulteil 10 sowie das feststehende Maulteil 3 wirken durch Verschieben des Schaftes 9 in der Art einer Stanze zusammen und bilden gemeinsam das distale Arbeitsende (Maul). Hierzu weisen die Maulteile 3, 10 einander zugewandte Schneidflächen/Stanzflächen 11, 12 auf.

In den Fig. 1 und 2 ist das Schiebeteil 9 in seiner Arbeitsstellung gezeigt. In der Arbeitsstellung ist das Schiebeteil 12 längsverschieblich am Schaft 2 geführt. Als Führung 13 ist im Bereich des distalen Endbereiches des Schaftes 2 eine Führungsnut 14 vorgesehen, in die ein unterer Führungsnuteingriffsabschnitt 15 im Bereich des distalen Endbereichs des Schiebeteils 9 eingreift. Die Führung 13 ist so konzipiert, dass das Schiebteil 9 in seiner Arbeitsstellung über den gesamten Längsverschiebeweg entlang des Schaftes, also auch in einer distal ausgelenkten und in einer proximal ausgelenkten Endposition, die den Schwenk- bzw. Öffnungswinkel der Maulteile 3, 10 zueinander definieren, in der Führung 13, genauer der Führungsnut 14 gefangen ist.

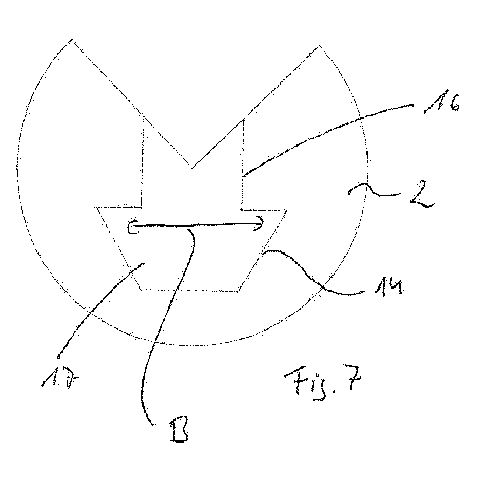

Im Hinblick auf eine bevorzugte Ausgestaltung der Führungsnut 14 und der Führung 13 wird auf Fig. 7 verwiesen.

Zu erkennen ist hier eine querschnittliche Darstellung des Schaftes 2. In diesem befindet sich die nach oben offene Führungsnut 14, die als hinterschnittene Nut ausgebildet ist.

Die Führungsnut 14 ist nicht klassisch als T-Nut ausgebildet, sondern ein unterhalb einer Führungsnutöffnung 16 angeordneter Hinterschnittraum 17 weist eine trapezförmige Querschnittskontur auf. Hierzu ist der Hinterschnittraum 17 in Richtung der Breitenerstreckung B des Schaftes 2 in einem an die Führungsnutöffnung 16 angrenzenden, hier oberen, Bereich, breiter ausgestaltet als in einem weiter davon beabstandeten (unteren) Bereich, also im Bereich des Nutgrundes. Die Seitenwände der Nut sind abgeschrägt, so dass sich die Trapezform ergibt. Hierzu ist im Bereich seitlich bzw. in Richtung der Breitenerstreckung B benachbart zum Hinterschnittraum vergleichsweise viel Material (Stahllegierung) vorhanden, so dass insgesamt hohe Führungskräfte aufgenommen werden können, ohne eine Beschädigung des Schaftes 2 zu riskieren.

Um eine erleichterte Reinigung des chirurgischen Instrumentes zu erreichen, ist eine Drehachse 18 für das Schiebeteil 9 vorgesehen. Ein Verschwenken um diese Drehachse 18 ist in der in den Figuren 1 und 2 gezeigten Arbeitsstellung, in der das Schiebeteil 2 in der Führung 13 gefangen ist jedoch nicht möglich. Wie sich aus den Figuren ergibt, durchsetzt die beispielhaft von einer Kopfschraube 19 oder alternativ eine Kopfniete gebildete Drehachse 18 ein Langloch 20 im Schiebeteil 9, so dass das Schiebeteil 9 relativ zu der Drehachse 18 längsverschieblich ist. Die Kopfschraube 18 ist fest relativ zum Schaft 2 positioniert.

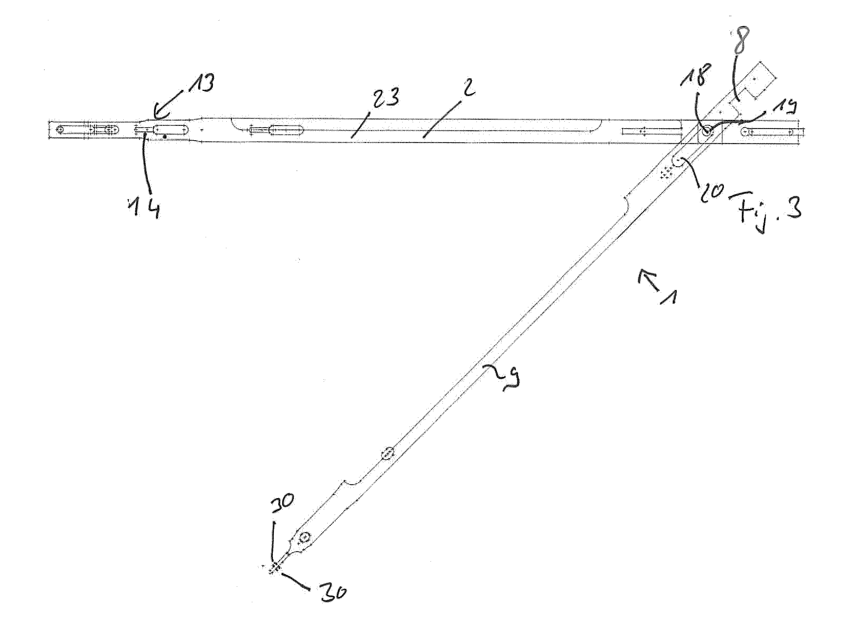

Dem Schiebeteil 9 sind Verriegelungsmittel 21 zugeordnet, die das Schiebteil 9 in der in Fig. 1 dargestellten Arbeitsstellung sichern, in der es ausschließlich translatorisch relativ zu dem Schacht 2 verstellbar ist und zwar durch Verschwenken des beweglichen Griffteils 6, um das Maul 22, welches von den Maulteilen 3, 10 gebildet ist, zu öffnen und zu schließen. Die Verriegelungsmittel 21 begrenzen hierzu den maximalen Schwenkwinkel des beweglichen Griffteils. Durch Betätigen bzw. Entriegeln der Verriegelungsmittel 21 wird der maximale Verschwenkwinkel vergrößert, wodurch das Schiebeteil 9 aus der Führung 13 freikommt, bzw. in eine Reinigungsstellung (vgl. Fig. 3) verstellbar ist, in der es um die Drehachse 18, welche senkrecht zur Schwenkachse 5 sowie senkrecht zur Längserstreckung des Schaftes 2 orientiert ist, verschwenkbar ist.

Fig. 3 zeigt das chirurgische Instrument in seiner Reinigungsstellung mit verschenktem Schiebeteil 9. Zu erkennen ist, dass in diesem verschenkten Zustand eine Schaftoberseite 23, die ansonsten von dem Schiebteil 9 verdeckt ist, optimal reinigbar ist. Das Gleiche gilt für eine in Fig. 3 nicht zu erkennende, der Schaftoberseite 23 zugewandte Schiebeteilunterseite.

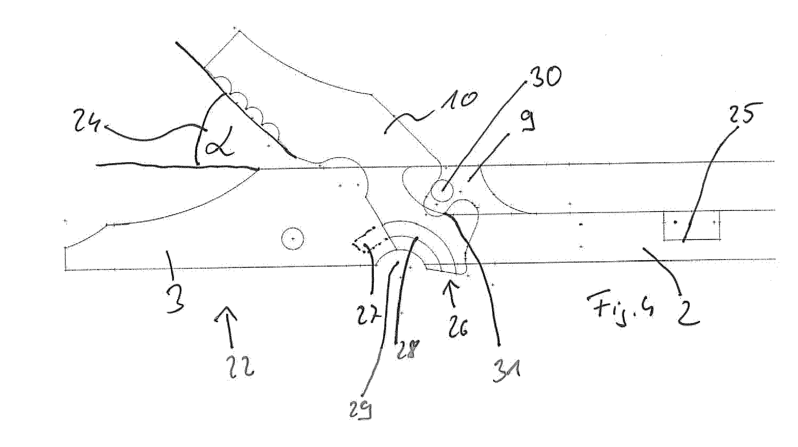

Um ein Verschwenken des Schiebeteils 9 zu ermöglichen, muss dieses, wie später noch erläutert werden wird, werkzeuglos von dem bewegbaren Maulteil 10 entkoppelt werden. Gleichzeitig ist die Aussparung 8 zusammenwirkend mit dem verschwenkbaren Griffteil, wie sich aus Fig. 3 ergibt seitlich offen ausgestaltet, um aus dem Wirkbereich des verschwenkbaren Griffteils 6 seitlich weg verschwenkt werden zu können. Auch ist proximal zur Führungsnut, wie sich aus Fig. 4 ergibt, im Schaft 2 ein seitlich offenes Fenster 25 vorgesehen, durch welches heraus der trapezförmig konturierte Führungsnuteingriffsabschnitt des Schiebteils 9 seitlich heraus verschwenkbar ist.

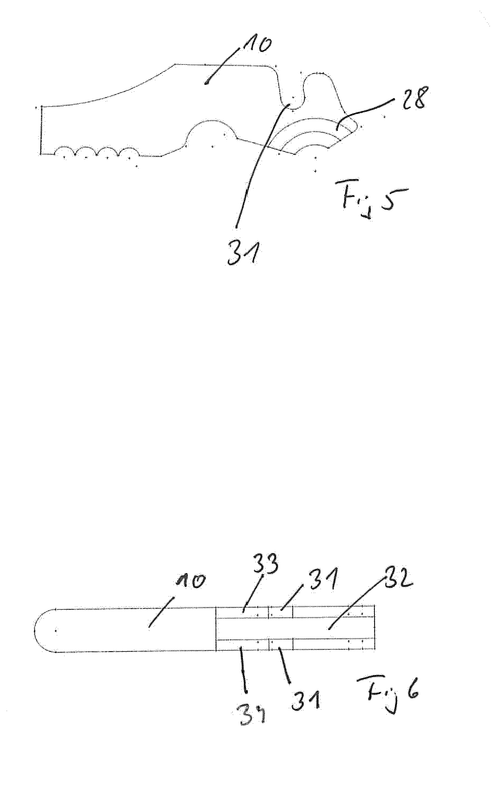

Im Folgenden wird anhand der Figuren 4 bis 5 die lösbare Kopplung zwischen Schiebeteil 9 und beweglichem (verschwenkbarem) Maulteil 10 sowie die Lagerung des Maulteils 10 am Schaft 2 im Detail erläutert.

Wesentlich ist auch, dass das bewegliche Maulteil 10 über eine Kulissenführung 26 am Schaft 2 geführt ist. Die Kulissenführung 26 umfasst zwei entlang der Breitenerstreckung des Schaftes 2, im Schaft ausgebildete teilkreisförmig gekrümmte Kulissennuten 27, innerhalb derer durch Verstellen des Schiebeteils 9 das bewegliche Maulteil 10 mittels eines einteilig mit dem beweglichen Maulteil 10 ausgebildeten Eingriffsabschnittes verschiebbar ist. Der Eingriffsabschnitt kann also innerhalb der teilkreisförmigen Führungsnut 27 verschoben werden und ist dadurch um eine gedachte Schwenkachse 29 verschwenkbar, relativ zu dem feststehenden Maulteil 3. Konkret sind an dem beweglichen Maulteil 10 zwei in einander entgegengesetzte Breitenrichtungen vorstehende, formkongruent zur Kulissennut 27 gekrümmte Eingriffabschnitte 28 vorgesehen, die in die jeweils dem zugehörigen Eingriffabschnitt 28 zugewandte bzw. offene Kulissennut 27 des Schaftes 2 eingreifen, wodurch eine großflächige Kraftaufnahme gewährleistet ist. Bei einer Verschwenkbewegung des beweglichen Maulteils wandert dessen Eingriffsabschnitt entlang der Kulissennut und legt bei dieser Bewegung auch eine translatorische Bewegungskomponente (Vektor) zurück.

In der Arbeitsstellung ist, wie erwähnt, das Schiebeteil 9 gekoppelt bzw. wirkverbunden mit dem beweglichen Maulteil 10 und zwar über zwei seitlich bzw. in Richtung der Breitenerstreckung des Instrumentes vorstehende Stiftfortsätze 30, denen jeweils eine Gelenkvertiefung 31 zur Bildung von Schwenkgelenken zugeordnet ist. Hierzu ist in dem verschwenkbaren Maulteil 10 eine nach distal offene Aussparung 32 vorgesehen, in die das Schiebteil 9 in seinem distalen Endbereich in seiner Arbeitsstellung eingreift, derart, dass die Stiftfortsätze 30 in jeweils einer Gelenkvertiefung 31 aufgenommen ist. Die Gelenkvertiefungen 31 sind in parallelen Wandabschnitten 33, 34 des bewegbaren Maulteils 10 aufgenommen, die die Aussparung 32 seitlich begrenzen.

In Fig. 4 ist ein maximaler Öffnungswinkel α des Mauls 22 bei in der Arbeitsstellung befindlichem Schiebeteil 9 gezeigt. D.h. eine weitere translatorische Verstellbewegung des Schiebteils 9 zu einem weiteren Öffnen des Mauls 22 wird durch die Verriegelungsmittel verhindert. Nach Entriegeln der Verriegelungsmittel kann das Schiebeteil 9 weiter in der Zeichnungsebene nach rechts, d.h. nach proximal bewegt werden wodurch sich dann ein Entriegelungswinkel der Maulteile 3, 10 zueinander einstellt, der größer ist als der maximale Öffnungswinkel 24 (α) in der Arbeitsstellung. Hierdurch können dann die Stiftfortsätze 30 nach proximal aus der jeweils zugehörigen Gelenkvertiefung 31 selbsttätig herausbewegt werden, wodurch das Schiebeteil freikommt.

Gemäß einer bevorzugten, hier realisierten Ausführungsform ist das Schiebteil 9 nicht unmittelbar nach Entriegeln der Verriegelungsmittel verschwenkbar, sondern muss hier noch ein Stück weit in proximaler Richtung verschoben werden, zum einen um aus den Gelenkvertiefungen 31 freizukommen und, was wesentlich ist, auch freizukommen aus der Führungsnut 14, was dann erreicht ist, wenn der lediglich beispielhaft trapezförmige Führungsnuteingriffsabschnitt des Schiebteils 2 seitlich fluchtet mit dem Fenster 25 im Schaft 2.

### Bezugszeichenliste

- 1: chirurgisches Instrument
- 2: Schaft
- 3: feststehendes Maulteil
- 4: proximales Griffteil
- 5: Schwenkachse
- 6: verschwenkbares Griffteil
- 7: Ende
- 8: Aussparung
- 9: Schiebeteil
- 10: bewegbares Maulteil
- 11: Stanzfläche
- 12: Stanzfläche
- 13: Führung
- 14: Führungsnut
- 15: Führungsnuteingriffsabschnitt
- 16: Führungsnutöffnung
- 17: Hinterschnittraum
- 18: Drehachse
- 19: Kopfschraube
- 20: Langloch
- 21: Verriegelungsmittel
- 22: Maul
- 23: Schaftoberseite
- 24: Öffnungswinkel
- 25: Fenster
- 26: Kulissenführung
- 27: Kulissennut
- 28: Eingriffabschnitt
- 29: Schwenkachse
- 30: Stiftfortsätze
- 31: Gelenkvertiefungen
- 32: Aussparung
- 33: Wandabschnitt
- 34: Wandabschnitt

## Patentansprüche

1. Chirurgisches Instrument, insbesondere Arthroskopie-Stanze, mit einem Maul (22), umfassend ein feststehendes Maulteil (3) und ein relativ zu dem feststehenden Maulteil (3) durch Verschenken eines verschwenkbaren Griffteils (6) in einer Kulissenführung (26) entlang einer gekrümmten Führungsbahn zwischen einer Schließposition und einer Offenposition verschiebbares bewegliches Maulteil (10),
**dadurch gekennzeichnet,**
**dass** das chirurgische Instrument (1) als Schiebeschaftinstrument ausgebildet ist, umfassend einen dem feststehenden Maulteil (3) zugeordneten, bevorzugt einteilig mit diesem ausgebildeten, Schaft (2) und ein durch Verschwenken des Griffteils (4, 6) relativ zu dem Schaft (2) translatorisch entlang des Schaftes (2) langsverschiebbares Schiebeteil (9), welches zwischen einer Arbeitsstellung, in der es an dem Schaft (2) längsverschieblich in einer Führung (13) geführt und gelenkig mit dem beweglichen Maulteil (10) zum Verstellen des Mauteils (3, 10) zwischen der Schließposition und der Offenposition bei einer geführten Verschiebebewegung des Schiebeteils (9) gekoppelt ist und einer Reinigungsstellung, in der das Schiebeteil (9) von dem beweglichen Maulteil (10) entkoppelt, aus der Führung (13) befreit und um eine Drehachse (18) relativ zu dem Schaft (2) sowie relativ zu dem beweglichen Maulteil (3) verschwenkbar ist, verstellbar ist, und dass dem Schiebeteil (9) manuell betätigbare Verriegelungsmittel (21) zugeordnet sind, mit denen das Schiebeteil (9) in seiner Arbeitsstellung gegen ein unbeabsichtigtes Überführen in die Reinigungsstellung sicherbar ist.

2. Chirurgisches Instrument nach Anspruch 1,
**dadurch gekennzeichnet,**
**dass** ein senkrecht zur Längserstreckung des Schaftes (2) und vorzugsweise parallel zur Drehachse (18) gemessene Abstand des Schiebeteils (9) zum Schaft (2) in der Schließposition und der Offenposition des beweglichen Maulteils (10) sowie während der Verstellbewegung zwischen diesen Positionen gleich ist, oder dass das Schiebeteil (9) in der der Schließposition und der Offenposition des beweglichen Maulteils (10) sowie während der Verstellbewegung auf einer dem Schaftteil (9) zugewandten Seite, insbesondere Oberseite, des Schaftes aufliegt.

3. Chirurgisches Instrument nach einem der Ansprüche 1 oder 2,
**dadurch gekennzeichnet,**
**dass** bei parallel zueinander ausgerichtetem Schiebeteil (9) und Schaft (2) das Schiebeteil (9) nach Entriegeln der Verriegelungsmittel (21) durch Verstellen des Schiebeteils (9) mittels des verschwenkbaren Griffteils (6) von dem Maul (22) weg von dem bewegbaren Maulteil (10) entkoppelbar und/oder durch Verstellen des Schiebeteils (9) mittels des verschwenkbaren Griffteils (6) in Richtung Maul (22) mit dem bewegbaren Maulteil (10) gelenkig koppelbar ist.

4. Chirurgisches Instrument nach einem der vorhergehenden Ansprüche,
**dadurch gekennzeichnet,**
**dass** die Kulissenführung (26) derart ausgebildet ist, dass das bewegliche Maulteil (10) während seiner Verschiebebewegung entlang einer Teilkreisbahn um eine gedachte Schwenkachse (29) verstellbar ist.

5. Chirurgisches Instrument, nach einem der vorhergehenden Ansprüche,
**dadurch gekennzeichnet,**
**dass** die Kulissenführung (26) mindestens eine, insbesondere schaftseitige und/oder feststehende maulteilseitige, gekrümmte, die Führungsbahn definierende Kulissennut (27) umfasst, in der ein, insbesondere gekrümmter, bevorzugt beweglicher maulteilseitiger, Eingriffsabschnitt (28) entlang der Kulissennutlängserstreckung bei der Bewegung des bewegliches Maulteils (10) zwischen der Schließposition und der Offenposition verschiebbar ist.

6. Chirurgisches Instrument nach einem der vorhergehenden Ansprüche,
**dadurch gekennzeichnet,**
**dass** das Schiebeteil (9) zwei seitliche, ineinander entgegengesetzte Richtungen weisende, senkrecht zur Längsachse des Schaftes (2) orientierte Stiftfortsätze (30) aufweist, die in jeweils eine zugehörige offene Gelenkvertiefung (31) des beweglichen Maulteils (10) eingreifen.

7. Chirurgisches Instrument nach einem der vorhergehenden Ansprüche,
**dadurch gekennzeichnet,**
**dass** das Schiebeteil (9) in seiner Arbeitsposition entlang der Längserstreckung des Schaftes (2) in eine in Richtung proximalem Instrumentenende offene Aussparung (32) ragt, die von zwei entlang einer senkrecht zur Längserstreckung des Schaftes (2) verlaufenden Querrichtung beabstandeten Wandabschnitten (33, 34) begrenzt ist.

8. Chirurgisches Instrument nach einem der vorhergehenden Ansprüche,
**dadurch gekennzeichnet,**
**dass** das bewegliche Maulteil (10) mit dem feststehenden Maulteil (3) in der Offenstellung einen Öffnungswinkel (α), insbesondere von kleiner 90°, aufspannt, der, insbesondere mittelbar, von den Verriegelungsmitteln (21) begrenzt ist, und dass das bewegliche Maulteil (10), bevorzugt nach Entriegeln der Verriegelungsmittel (21), zum Entkoppeln von dem Schiebeschaft in eine Entriegelungsposition verstellbar ist, in der das bewegliche Maulteil (10) und das feststehende Maulteil (3) einen Entriegelungswinkel aufspannen, insbesondere von größer 90°, der größer ist als der Öffnungswinkel.

9. Chirurgisches Instrument nach einem der vorhergehenden Ansprüche,
**dadurch gekennzeichnet,**
**dass** die Führung (13) zur längsverschieblichen Führung des Schiebeteils (9) am Schaft (2) eine hinterschnittene Führungsnut (14), insbesondere im Schaft (2), aufweist, mit einer sich in Richtung der Schaftlängserstreckung erstreckenden Führungsnutöffnung (16), und dass benachbart zu der Führungsnutöffnung (16) ein Hinterschnittraum(17) ausgebildet ist, in dem in der Arbeitsstellung ein Führungsnuteingriffsabschnitt (15), insbesondere des Schiebeteils (9), längsverschieblich geführt ist und dessen senkrecht zur Schaftlängserstreckung sowie senkrecht zur Führungsnuttiefe gemessene Hinterschnittraumbreite in Richtung der Führungsnuttiefenerstreckung weiter von der Führungsnutöffnung (16) beabstandeten Bereich kleiner ist als in einem näher an der Führungsnutöffnung (16) gelegenen Bereich.

10. Chirurgisches Instrument nach Anspruch 9,
**dadurch gekennzeichnet,**
**dass** eine Querschnittskontur des Hinterschnittraums (17) und/oder des Führungsnuteingriffsabschnittes (15) trapezförmig ist.

11. Chirurgisches Instrument nach einem der vorhergehenden Ansprüche,
**dadurch gekennzeichnet,**
**dass** das bewegliche Maulteil (10) und der Schaft (2) sowie das feststehende Maulteil (3) jeweils aus einer Stahllegierung ausgebildet sind, wobei die Stahllegierung des Schaftes (2) und des feststehenden Maulteils (3), insbesondere eine 40.34 Stahllegierung, einen höheren, insbesondere um mindestens 0,2 % höheren, Kohlenstoffanteil und/oder eine um mindestens 5 HRC höhere Rockwellhärte aufweist als die Stahllegierung, insbesondere eine 40.21 Stahllegierung, des Schaftes (2) und/oder des feststehenden Maulteils (2).

12. Chirurgisches Instrument nach einem der vorhergehenden Ansprüche,
**dadurch gekennzeichnet,**
**dass** das Schiebeteil (9) nach Entriegeln der Verriegelungsmittel (21) translatorisch weiter von dem Maul (22) weg verstellbar ist, insbesondere derart, dass das Schiebeteil (9), bevor dieses aus der Führung (13) frei kommt, um relativ zu dem Schaft (2) und dem Maul (22) verschwenkt werden zu können, ein Stück weit innerhalb der Führung (13) gefangen translatorisch von dem Maul (22) weg verstellbar ist, insbesondere über eine Strecke von mindestens 1 mm, bevorzugt zwischen 1 mm und 5mm, ganz besonders bevorzugt zwischen 2mm und 3mm.

13. Chirurgisches Instrument nach einem der vorhergehenden Ansprüche,
**dadurch gekennzeichnet,**
**dass** die Drehachse (18), um die das Schiebeteil (9) in der Reinigungsstellung verschwenkbar ist, senkrecht zur Schwenkachse (5) des Griffteils (4, 5) orientiert ist sowie senkrecht zur Längserstreckung des Schaftes.

14. Chirurgisches Instrument nach einem der vorhergehenden Ansprüche,
**dadurch gekennzeichnet**
**dass** der Schiebeschaft sowohl in der Arbeitsteilung als auch in der Reinigungsstellung, während der Überführung von der Arbeitsstellung in die Reinigungsstellung sowie während der Verschwenkbewegung unverlierbar am Schaft (2) gesichert ist.
